# EUROPEAN PATENT APPLICATION

(11) **EP 4 105 816 A1**
(43) Date of publication of application: **21.12.2022**
(21) Application number: 21179618.0
(22) Date of filing: 15.06.2021
(51) Int. Cl.: G06K 9/00

(54) **METHOD AND APPARATUS FOR INSPECTING, COUNTING AND DISPENSING ITEMS**

(71) Applicant: Data Detection Technologies Ltd., 9980300 Kibbutz Tzora (IL)
(72) Inventor: ADAM, Amichai, 9980300 Kibbutz Tzora (IL); MALUL, Pinhas, 9980300 Kibbutz Tzora (IL); ADADA, Eliad, 9980300 Kibbutz Tzora (IL); NUCHAM, Sorin, 9980300 Kibbutz Tzora (IL)
(74) Representative: Hoyng Rokh Monegier B.V.

(57) **Abstract**

There is provided a method and apparatus for counting items and identifying rogue of deviant items, for example in bulk of falling items.

## Description

### Field of the invention

The present invention relates to a method and apparatus for dispensing a multiplicity of discrete items into groups (or "batches"), each group containing a predetermined number of the items. In particular the invention concerns a method and apparatus for rapidly yet precisely dispensing a predetermined number of discrete items into batches while also inspecting one or more qualities of the discrete items.

### Background of the invention

It is frequently required to dispense discrete items, into batches of precise quantity. Examples include dispensing medicinal tablets, pills, capsules, seeds, candies or the like into bottles, sacks or other containers. Common nomenclature may refer to devices or systems for doing this as counting machines or dispensing machines.

In some dispensing tasks, the finished container must not contain less than the predetermined number of items. For example, when dispensing certain pills, a full treatment cycle for a patient may have to be provided, therefore at least the predetermined number of items must be provided in each container.

On the other hand, dispensed items may be expensive if too many of the containers contain more than the predetermined number of items it translates to direct loss to the supplier of the items or to the packing organization.

In particular, the pharmaceutical primary packaging industry is characterized by strict regulations and demands. High levels of accuracy are needed, and undercounting or overcounting should be avoided, and preferably excluded.

U.S. Patent No. 5,473,703 to Smith, entitled "Methods and apparatus for controlling the feed rate of a discrete object sorter/counter", discloses a controller which adjusts a vibration frequency of a feed bowl dependent upon the passage of sensed and counted objects, to provide control over the number of objects dispensed.

U.S. Patent No. 6,659,304 to Geltser et al., entitled "Cassettes for systems which feed, count and dispense discrete objects", discloses a high capacity cassette for an object counting and dispensing system, that includes, inter alia, a structure which feeds the discrete objects in single file toward an exit hole.

U.S. Patent No. 6,449,927 to Hebron et al., entitled "Integrated automated drug dispenser method and apparatus", discloses, inter alia, singulation control, which is a process by which drugs move through a canister in a nearly single-file fashion. Hebron discloses that in order to minimize fill time, a drive frequency is increased slowly to approach a maximum detection rate of a sensor. For accuracy of count, the final few items in a dispensed batch may be dispensed more slowly than the foregoing majority.

European Patent Application No. 1,852,372 to Ogawa et al., entitled "Vibrating bowl, vibrating bowl feeder, and vacuum deposition apparatus", discloses, inter alia, a vibrating bowl and the like, which are capable of accurately counting the number of objects to be fed, accurately leading objects one by one to an external place per unit time, and aligning collectively of objects into a row or tier at an intermediate point on a feed passage by simple alignment means.

U.S. Patent Application Publication No. 2003/022291 to Gerold et al., entitled "Automated pill-dispensing apparatus", discloses, inter alia, a bulk storage unit useful for automatically dispensing solid pills includes a track having a length, an upstream end and a downstream end, the track being adapted to feed pills along its length in a longitudinal direction when the track is vibrated.

U.S. Patent Application Publication No. 2010/0205002 to Chambers, entitled "Automated pill-dispensing apparatus", discloses, inter alia, that pills advance up a spiraling edge of a vibratory feeding bowl and pass through a singulator.

U.S. Patent No. 6,449,927 to Ishizuka, entitled "Automatic high-speed pill counting apparatus", discloses, inter alia, an apparatus comprising a cylindrical pill hopper having a pill exit and a center hole in a base plate; a rotational separative feeder mounted in the cylindrical pill hopper and relies upon a complex alignment of moving parts.

U.S. Patent No. 4,382,527 to Lerner, entitled "Article handling system with dispenser", discloses, inter alia, a system for dispensing weighed or counted articles fed from a supply hopper by a vibratory conveyor to maintain a controlled level of articles in a bowl-shaped feeder hopper.

Japanese Patent No. 2,132,011 to Kazumi et al., entitled "Granular material discharging device", discloses, in its published English abstract, improvement of the discharge control precision by selection of vibration frequency in response to a load change.

Some dispensing and packing machines include a counting mechanism for determining the actual number of collected objects. By monitoring objects interrupting the illumination of a light source onto a pixelated array, it is possible to count objects being poured.

In U.S. Patent 5,768,327 to Pinto et al., entitled "Method and apparatus for optically counting discrete objects", there is described an object counter including a feeding funnel having a frustroconical section, the narrow end of which is coupled to a substantially vertical feeding channel having a substantially rectangular cross section. A pair of linear optical sensor arrays are arranged along adjacent orthogonal sides of the feeding channel and a corresponding pair of collimated light sources are arranged along the opposite adjacent sides of the feeding channel such that each sensor in each array receives light the corresponding light source. Objects placed in the feeding funnel fall into the feeding channel and cast shadows on sensors within the arrays as they pass through the feeding channel.

U.S. Patent No. 5,317,645 to Prozek et al., entitled "Method and apparatus for the recognition and counting of discrete objects", discloses, inter alia, an apparatus for counting discrete objects of various sizes and shapes as they travel through the apparatus in a disorderly flow. The apparatus includes a sensor array which comprises a plurality of photodetectors arranged in a linear fashion. The discrete objects are passed over the sensor array. By utilizing the sensor array as a means for obtaining information about the discrete objects, the apparatus samples the sensor array at predetermined time intervals, examines the various contours of the images produced through the sampling and based upon predetermined criteria determines whether an image represents one or more objects.

Bulk-counting machines as also known, such as are described in European patent EP2600288 B1, which addresses a number of issues by providing a system and method for counting objects like medical tablets that free-fall from an open end of a conveyor belt carrying and transporting the objects in bulk. The machine makes use of digital images acquired by cameras to count falling objects. The proposed systems and methods can bulk count falling items while inspecting one or more qualities of the discrete items so that deviant or rogue items are excluded.

For example, in particular when handling medicinal tablets, pills or capsules, a single dispensing machine may be consecutively used for the handling and dispensing of batches of difference medicines. In such a situation it may be important to ensure that stray or unnoticed medicinal product (tablets, pills, capsules etc.) lingering in an apparatus from a previously handled product variety (rogue items) are not entrained into a batch of medicines handled as part of subsequent variety. This could result in provision of incorrect and/or too little of a particular medicament being provided to an end user.

The pharmaceutical primary packaging industry is characterized by strict regulations and demands. Cross contamination is strictly forbidden.

In that respect, counting and dispensing machines may be employed for dispensing various types of medicines at different times. It is conceivable that 30, 40 or an even greater variety of medicines at may be processed by a single machine at different times, raising the risk of cross-contamination between varieties if clean-up is not perfect for all system machinery upon switching from handling one variety to a subsequent variety.

Similar concerns may arise when dispensing other objects such as seeds, diamonds, or conceivably any bulk supplied product, requiring both accurate numerical dispensing but also accurate qualitative dispensing.

Attempts have been made in the prior art to mitigate the above concerns. For example, when incorporated into a greater whole, the known counting systems may be combined with color analysis modules for identifying rogue or stray pills or tablets based on color. For example, pharmaceuticals are often color coded for ease of identification by a user or medical personnel. This same quality may be used by a dispensing machine to identify 'incorrectly' colored items (items from a previous operation) within a batch, by visual inspection.

A number of systems are known that, in addition to counting and controlling the number of dispensed items, assess additional qualities of the dispensed items by using an external inspection system, for example.

Multi-channel counting machines typically comprise several generally parallel, horizontal channels carrying and transporting medical tablets or capsules in single file . An inspection system can be arranged above the horizontal channels and utilizes cameras inspecting tablets or capsules in real-time before being bottled. Image analysis techniques are used to examine the physical shape of the objects, for example whether broken or undersized.

Slat-counting machines adapt a matrix of slats or physical housings instead of channels for carrying and transporting objects like pills. Each slat securely carries a single pill, and the matrix of slats provides a fixed and predictable display of the pills for imaging. An inspection system can be arranged above so that Each pill is inspected before being bottled. these inspection machines typically comprise a detection unit and a lighting arrangement that is installed above the moving slats and utilize LED-based technology to physically examine the pills in 2D. Accordingly, the physical pill shape can be examined for size, rogue and intactness.

The need to feed and separate the objects into multiple channels or slats significantly limits the dispensing process with regards to its industrial throughput.

Furthermore, it has been identified by the inventors that the prior art machines may suffer from a lower accuracy in color identification on account of background color and/or dust or powder that may result from handling of the items, especially uncoated or non-sealed pills. It has been identified by the inventors that attrition during handling can result in a background layer of similarly colored powder being created and complicating image analysis.

Furthermore, it has been identified by the inventors that identification of rogue or deviant items in the prior art machines may result in undesirable slowing of feeding rate due to limited capabilities of sampling rates

In addition to the foregoing, systems for identification and removal of deviant items based on qualities such as luminance of reflected light are also known. In European Patent No. 1,083,007 to Satoru at el., entitled "Method and apparatus for sorting granular objects with at least two different threshold levels", discloses, inter alia, a method and system for sorting items in different sizes, wherein granular objects flowing in a continuous form are irradiated by light. The resulting image element signals from a solid-state image device are analysed for luminance brightness and dark tone of color to detect a defective portion of a granular object (e.g. a darkened portion in a grain of rice). A bulk-stream of rice is allowed to flow by gravity along a chute, past an optical detection section. Upon detection of a deviant rice grain, air jets are applied to blast the deviant grain(s) out of the stream. Counted batches, let alone accurately counted items per batch, are not achieved by such a system.

While counting and dispensing systems and methods have previously been proposed and employed in the technical field, the needs of speedy yet accurate numerical dispensing and qualitatively accurate dispensing, can still be better addressed.

The present invention may assist in addressing the needs of quality and characteristics control, and/or ensuring accurate counting of pharmaceuticals, preferably in an efficient and effective manner. In particular counting and inspection capabilities are currently complex, inefficient, and expensive, based on single file counting. Such counting requires mechanical object separation and are not able to recognize clustered objects.

### Summary of the invention

There is provided, in accordance with an embodiment, a method comprising:
- allowing items to fall, preferably free-fall, into a volume;
- employing a digital imaging device to acquire images of the items while said items are falling toward the volume, preferably wherein acquired images comprise imaging of multiple items per image;
- processing the images to count each item in free-fall;
- processing the images to determine at least one characteristic, preferably a color, of each falling item, and
- evaluating said determined characteristic in relation to at least one predetermined characteristic.

In a preferred embodiment the method may optionally be implemented for dispensing a plurality of discrete items to a collection volume, the method further comprising the step of stopping dispensing to the collection volume once a predetermined number of counted items, forming a batch, is collected.

The items may preferably fall into the volume as a bulk. That is, more than one item, preferably more than two, three or four items fall simultaneously toward the volume in parallel. This is distinct from a single-file dispensing of items in which substantially every dispensed item would be vertically spaced from prior and subsequently dispensed items. Bulk dispensing provides dispensing of multiple items so as to fall vertically simultaneously, with items being simultaneously at a similar overlapping height as they fall. Horizontal spacing or separation normal to the falling direction is preferred yet not required. Such bulk dispensing may be understood as a "water-fall" like flow of items from the dispenser, as opposed to a single-line flow of items. The invention may also or alternatively be implemented for single-file dispensing of items in which substantially every dispensed item is vertically spaced from prior and subsequently dispensed items.

The digital imaging device acquires multiple images, said images being of multiple falling items. That is, acquired images capture image date of multiple items per acquired image, preferably multiple items across an x-axis of a captured image. This may be achieved by provision of the bulk dispensing of items in which said items fall in parallel, and provision of a suitably angled lens, such as a wide-angle lens, to image the area through which items fall.

It may be considered advantageous that both the counting and the item-characteristic checking are carried out by a single module or analysis point within a process or apparatus. This may be advantageously contrasted to systems in which counting and inspection capabilities employ two separated systems; i.e. a module for counting and a module for inspection. Such dual systems may then require multiple and distinct communication protocols, algorithm processing, multiple interfaces as well as physical components, possibly resulting with low efficiency, high costs and high operation complexity

The present invention may help to address such concerns through providing item counting and item-characteristic inspection using the same image set, same camera system, same operation and/or the same user interface. This may assist in improving processor efficiencies, possibly improving real-time analysis.

The method of the invention may be implemented for counting and inspecting of objects fed as bulk material, having a feeding device that conveys objects in bulk to a counting and inspection digital imaging device, wherein the imaging device is designed for contactless counting and inspection of multiple objects while the objects are in free fall.

In a preferred embodiment there is provided, in accordance with an embodiment, a method for dispensing a batch of a predetermined number of discrete items to a collection volume, the method comprising:
- providing a conveyor with an open dispensing end;
- transporting items in bulk on the conveyor to the open dispensing end of the conveyor;
- allowing items to free-fall from the open dispensing end of the conveyor into a collection volume;
- employing a digital imaging device to acquire images of the items during free-fall toward the collection volume;
- processing the images to count each item in free-fall;
- processing the images to determine at least one characteristic, preferably a color, of each free-falling item,
- evaluating said determined characteristic in relation to at least one predetermined characteristic; and
- stopping dispensing to the collection volume once a predetermined number of counted items forming a batch is collected.

In another aspect of the invention there is provided an apparatus for inspecting and dispensing items, the apparatus comprising:
a dispenser comprising an open dispensing end, configured to dispense a plurality of items, optionally in bulk;
a volume below the open dispensing end;
at least one digital imaging device positioned to capture images of items falling, preferably, free-falling from the open dispensing end, more preferably wherein arranged to acquire images comprising imaging of multiple items per image;
a processer configured to analyse images captured by the digital imaging device, said analysis comprising at least a count of the number of items, and a determination of at least one characteristic, preferably color, of each imaged item.

In a preferred embodiment the dispenser comprises a conveyor for dispensing items. The conveyor is provided with an open dispensing end, said conveyor is configured to transport items in single file or in bulk, preferably bulk, to its open end.

A conveyor is a device that moves, motivates otherwise displaces items from one location to another. A variety of conveyor systems are known in the art, preferred examples may include belt conveyors and vibratory conveyors (e.g. vibration plates), although gravity conveyance, pneumatic conveyors and other conveyors may be envisaged for use.

In a preferred embodiment, the apparatus further comprises a dispensing unit configured to dispense a predetermined number of counted objects.

In a preferred embodiment, the apparatus further comprises a supply unit configured to supply containers, wherein each container receives the predetermined number of counted objects from the dispensing unit.

In a preferred embodiment of the invention there is provided an apparatus for inspecting and dispensing batches of items, the apparatus comprising:
a conveyor with an open dispensing end, said conveyor configured to transport a plurality of items, preferably in bulk, to its open end;
a collection volume below the open dispensing end;
at least one digital imaging device positioned to capture images of items free-falling from the open end of the conveyor;
a processer configured to analyse images captured by the digital imaging device, said analysis comprising at least a count of the number of items, and a determination of at least one characteristic, preferably color, of each imaged item.
a dispensing unit configured to dispense a predetermined number of counted objects; and
a supply unit configured to supply containers, wherein each container receives the predetermined number of counted objects from the dispensing unit.

In contrast to some prior art devices, it has been found by the inventors that determining at least one characteristic, preferably a color, of each item, while the items are free-falling from the open end of a conveyor, may under circumstances be advantageous for improving accuracy in identification of rogue or deviant products and/or rate of throughput for a counting and dispensing device and method. In addition, advantageous may also be emergent in cost-reduction for production and/or maintenance of counting and dispensing devices.

It may be considered an advantage of the invention that inspection of the item characteristic(s) takes place for the item as it is falling. An advantage may be that items are more readily analysed by imaging because clustering of items is reduced or eliminated during falling. For example, items clustered on a delivery track may tend to separate during falling from that track, allowing improved visual analysis of the items. This may be seen as an advantage compared to other systems requiring specialized on-track item separation systems and/or single-file provision or dispensing.

For example, determination of a characteristic such as color of an item during free-fall may advantageously improve accuracy in the determination of an items color, for example its hue. In one aspect, for a free-fall item it is possible that the free-fall item is spaced from surrounding solid surfaces, such as a background surface. This may ease image processing because there is a lower burden in distinguishing items from a background plate. For example, if items are analyzed while resting on a substantially horizontal plate, an image processor must determine whether data in an image is an item of the batch, a rogue item, or a part of the supporting plate; this may slow processing or lead to errors. The benefit may be especially emergent for background or supporting surface that may collect dust layers, which may be more or less similar than the items to the counted and analyzed, and which may change throughout the course of a dispensing program. Naturally, cleaning processes to remove dust may negatively affect a rate of processing, so that avoiding such a need may be an advantage.

It may be considered an advantage of the invention that inspection of the item characteristic(s) takes place for the item as it is falling. An advantage may be that sensitivity to dust is reduced or eliminated. For example, some counting systems might be based on items places on a horizontal track. A problem is that dust may accumulate making analysis of items against a dust (of similar color) background complex or inaccurate. For such systems, accumulated dust might need to be removed from tracks to preform object inspection and regain accuracy. Again, removal of dust and cleaning operations may slow processes and reduce efficiency or accuracy more generally.

While color is a preferred characteristic, other characteristics of items may also or alternatively be analysed, including polarization, fluorescence, texture, translucence, and other information, which may can be used to represent images of objects for identification and flaw detection.

In line with the above, it may be preferable that the items in free-fall are spaced from any solid surface by at least 1 cm, preferably by at least 5 cm, or more preferably more than 10 cm, excluding the open end of the conveyor.

In some embodiments, a vertical gap is provided between the end of the conveyor and an upper edge of the area, to ensure that an end surface of the conveyor is not recorded in the images.

In a preferred embodiment, the characteristic of the items that is analysed is the visible color of the free-falling items. The color may, in particular, be associated with particular medicaments by a pharmaceutical company and may be used as a basis for rejecting suspect or rogue items not matching a predetermined color or color spectrum. Preferably the color is identified by any one or more of hue (pure color or mix of colors), tint (mixture of pure color and white), shade (mixture of pure color and black), and/or tone (mixture of pure color with any neutral or grayscale color). The color may also be determined as a spectral pattern, or as a predetermined output from a digital camera sensor (e.g. a CCD sensor or a CMOS sensor) imaging a color.

The predetermined characteristic may be programmed by presentation of exemplary items to the imaging system. For example, AI may be implemented to determine acceptable boundaries of the predetermined characteristic within the context of an operating environment, based on exemplary items and/or exemplary rogue items.

In a preferred embodiment of the invention, a batch of counted items will be rejected from further processing, e.g. packaging, supply to further workstations or supply to a customer if one or more inspected free-falling items has a characteristic that is deviant from a required characteristic.

Analysis may be done based on algorithm analysis and identification of color in known color spaces.

The sensor may preferably be a digital image sensor (preferably CMOS or CCD) configured and/or adapted to operate as global shutter. This may assist in reducing external interference, skew and/or smear effects. A digital image sensor operating in global shutter mode may begin and end the exposure of the majority of, or all of, the sensor pixels simultaneously. A global shutter can allow capture of high-speed moving objects without distortion.

Exposure times for image capture are preferably minimal. This may assist in avoiding interference, skew and/or smear effects associated with image capture of fast falling.

The invention may provide a method for rapidly and accurately dispensing a predetermined number of discrete items that have been inspected for rogue content.

The conveyor may transport multiple items towards an imaging device, wherein the items are arranged in a single layer and at least some of the items are transported in parallel upon the conveyor. The conveyor may be a parallel transport conveyor configured to transport multiple items, in parallel, from a hopper to an open end of the conveyor.

The parallel transport conveyor may be configured to transport items out of its open end at a rate of at least 10, 20, 30, 40, 50, or more items per second.

The parallel transport conveyor may be configured to have a width suitable to carry at least 5 items adjacent one another perpendicular to the direction of travel. More preferably at least 10, or at least 20 items.

The conveyor may be a substantially planar surface that does not comprise separating channels between items transported in parallel. The conveyor may be any known conveyor, for example a belt conveyor or a vibratory plate.

Suitable conveyors are discussed in patent publication EP2600288, which are incorporated herein by reference.

It is preferred that the imaging device to captures images of an area or volume immediately below the open end of the conveyor, so that items falling off the conveyor are recorded in the images while in free-fall, but while still containing some order from the single-depth layering on the conveyor.

In some embodiments, the area or volume immediately below the open end of the conveyor extends to an effective portion of the width of the end of the conveyor, such that all of the items transported in parallel and falling off the end of the conveyor in parallel are recorded in the images.

Suitable placement of the imaging devices is discussed in patent publication EP2600288, which placement is incorporated herein by reference.

The images are preferably processed in real-time to allow continuous determination of the number of falling items and their characteristic, e.g. color. Real-time processing allows dynamic action to be taken to control the number of dispensed items and to determine a particular batch includes a deviant or rogue item.

In a preferred embodiment, the apparatus or method dispense the plurality of items to a collection volume. Collection volumes may be packages such as a bottles, jars, boxes, bags, or similar.

In a preferred embodiment, the apparatus or method are suitable for dispensing one more batches of one or more predetermined numbers of items.

Achieving the desired number of items in a batch may involve stopping the conveyor before the number of falling items has reached the predetermined full number for the batch and continuing to determine the number of falling items until items cease to fall off the conveyor inertially; and automatically dispensing an additional number of items, to complete the predetermined number of items for the batch. In some embodiments, the automatic dispensing of the additional number of items comprises operating a supplementary item dispenser, to dispense exactly the number of items needed for completing the predetermined number of items. The items supplied by the supplementary item dispenser are preferably also analysed for rogue or deviant items.

In some embodiments, the processing of the images to continuously determine the number of falling items comprises increasing an item count by one when an item exits the area, wherein an exit is determined when a top portion of an item appears in an image but is missing from a consecutive image.

In some embodiments, the processing of the images to continuously determine the number of falling items comprises increasing an item count by one when an item enters the area, wherein entrance is determined when a bottom portion of an item appears in an image but is missing from an immediately preceding image.

In some embodiments, the processing of the images to continuously determine the number of falling items comprises: tracking each item over consecutive images, from entering the area until exiting the area; and increasing an item count by one upon the exit of each tracked item, to prevent a miscount if occasional noise appears in one or more of the images.

The processor of the apparatus of the invention may comprise a counting and analysis device configured to process the images in real time, to continuously determine the number of falling items and the characteristic of each of the items.

The apparatus of the invention may further comprise one or more of; an actuator configured to control operation of the conveyor in accordance with actuator control commands; a computing platform configured to receive the number of falling items and details of the characteristic from said counting and analysis device, and to generate commands to start or stop said conveyor based on the number of falling items and a predetermined target number, and/or to generate rejection commands to reject batches determined to contain deviant or rogue items.

The apparatus may be provided with a rejection conduit for ejection of batches that contain items identified as deviant from a required characteristic. For example, the apparatus may be configured to reject a batch of medicinal pills if a required pill color is red, yet a blue item is detected within the items analysed as having fallen into that batch.

In some embodiments, said counting and analysis device comprises an image sensor and is configured to capture images using a predetermined number of pixel rows of said image sensor, optionally the predetermined number being less than a total number of sensor rows existing in said image sensor.

In some embodiments, said analysis device is configured to determine the number of falling items by analyzing a pattern of sensor pixels affected by a falling item in consecutive samples of the image sensor.

In some embodiments the apparatus comprises one or more light sources to illuminate items in free-fall. Preferably the illumination is white light illumination, however, other colors of illumination may be used for specific items and processing programs.

In some embodiments, the apparatus further comprises a lens assembly for focusing light reflected from the falling items onto the imaging device.

### Brief description of the drawings

Various aspects of the invention will be further explained with reference to embodiments shown in the drawings wherein:
FIG. 1A shows a schematic illustration of a first exemplary embodiment of a machine for dispensing items;
FIG. 1B shows a schematic illustration of a second exemplary embodiment of a machine for dispensing items;
FIG. 2 is a flowchart of steps in a method for operating a dispensing machine;
FIG. 3A is an exemplary embodiment of the lens, sensor and lightening equipment used for counting the items falling off a conveyor;
FIG. 3B is an exemplary embodiment of the lens, sensor and lightening equipment used for counting the items falling off a sliding area;
FIG. 4A shows an exemplary snapshot of items falling off the sliding area;
FIG. 4B is a schematic illustration of a multiplicity of sensor lines captured during the falling of three exemplary tablets; and
FIG. 5 shows a schematic illustration of the mode of operation of a conveyor and a supplementary item dispenser.

### Description of illustrative embodiments

It will be appreciated that for simplicity and clarity of illustration, where considered appropriate, reference numerals may be repeated among the figures to indicated corresponding or analogous elements or steps. In addition, numerous specific details are set forth in order to provide a thorough understanding of the embodiments described herein. However, it will be understood by those of ordinary skill in the art that the embodiments described herein may be practiced without these specific details. Furthermore, this description is not to be considered as limiting the scope of the embodiments described herein in any way, but rather as merely describing the implementation of the various embodiments described herein. The following is a description of various embodiments of the invention, given by way of example only and with reference to the drawings.

The following description relates to rapid, accurate and efficient dispensing of predetermined quantities of quality-inspected discrete items, such as seeds, gems, medicinal tablets, pills, capsules, candies or the like,.

A technical problem addressed by the disclosed method and apparatus relates to a situation in which it is required to dispense substantially identical items from a container into separate packages, each package containing the same predetermined number of items (batch sizes). The dispensing has to be done at high accuracy, such that packages containing fewer or more than the predetermined number or tolerance thereabout, of items are avoided. In addition, to the precise numerical dispensing of items it can also be important that each of the dispensed items in a counted batch has a specified characteristic and that deviant or rogue items are excluded.

One technical solution is the provision of an apparatus and method for dispensing a predetermined number of items, wherein substantially each, preferably each, item is inspected or checked to ensure that it has a desired characteristic or that it does not have a deviant or rogue characteristic.

The apparatus may include a feeder such as a hopper or a silo which can contain a large number of items in bulk, which are to be dispensed. The hopper releases the items onto a conveyor activated by an actuator, the actuator controlled by a computing platform. The conveyor may be a conveyor belt, a vibrating conveyor, a vibrating chute, a chute having changing inclination, or any similar means for transporting items along a path. In some embodiments, the items are released from the hopper in a free manner, such that multiple items can be released simultaneously or with minimal time difference, so that a second item begins to release before a first item has been fully released. As a result of the motion of the conveyor and/or its vibrations, the items become randomly arranged on the surface of the conveyor, in a single layer, and in a way that multiple items run in parallel (as opposed to single file).

The vibrations of the actuator may make the items vibrate on the conveyor, so that each of them eventually assumes a stable posture. For example, some items, such as medicinal tablets, may be shaped as straight circular cylinders, or a similar shape having at least one substantially planar face. The vibrations may cause the tablets to move such that any one of their planar sides is flat on the conveyor, wherein usually tablets do not lean on each other, but rather a full planar face of each tablet is placed on the conveyor's surface. Items that do not have a planar side may assume a relatively stable posture according to their shape, center of gravity and the like. For example, tubular capsules with rounded ends may lie with the tubular surface, as opposed to the head, on the conveyor. Being symmetrical, even if a capsule rotates about its tubular surface, it will have the same projection when viewed from above.

As items reach an open dispensing end of the conveyor they free-fall in ballistic trajectory off the conveyor into a container or a track or processing path that eventually leads to a container. As the items fall from the conveyor's open end, the gaps between them tend to increase, and items that were previously touching each other while on the conveyor tend to separate. Accordingly, in an embodiment, an imaging device continuously captures images of an area immediately below the end of the conveyor ("imaging area"), so that the free-falling items are depicted in the images while in trajectory, when the vast majority of items, if not all of them, are spread out and not in contact with one another. This can aid improved analysis of the items during computerized analysis of the captured images, for counting and analysis of other characteristics such as color (hue, tint, shade, and/or tone, preferably hue or shade).

In an alternative embodiment, the conveyor, which is generally horizontal, ends with a sloped sliding area. The items fall off the conveyor to the sliding area, slide across the area and at its end fall into the container being filled or into a track of processing path that leads to the container. The slope of the sliding area provides for substantially the same speed function of the items travelling along the sliding area, and thus for substantially the same falling speed of the items at the instance they leave the sliding area. It also provides for increased spacing between items, and for a substantially similar trajectory once leaving the sliding area, therefore providing a similar angle in relation to the imaging device.

The items are analysed (counted and analysed for at least one characteristic such as color, polarization, fluorescence, texture, or translucence, but preferably color) as they free fall from the conveyor edge. Once a predetermined number of items have fallen into the container, the conveyor is stopped. In some embodiments, the predetermined number is an undershoot, i.e., a lesser quantity of items than required for a full batch, since it is taken into account that after the conveyor has stopped, one or more items may still fall through the imaging area into the container by virtue of inertial forces. The item(s) falling after the conveyor has stopped are counted and analysed for characteristics as well, and the total number of items in the container is determined.

In an embodiment, the system may be configured such that even with the inertial fall, the total number of dispensed items is in almost all cases still fewer than the final required number. In these cases, the control system re-activates the conveyor in one or more pulses, as necessary, so that additional items fall off and complete the final number. Such a process is detailed in EP2600288, which process is incorporated herein by reference. Once the number of dispensed items has been reached (or exceeded) the number of required items, the container is removed, and a new container is placed and filled in the same manner.

In some embodiments, an additional device, referred to as a supplementary item dispenser, may be used to complete the items still missing from the final number. The supplementary item dispenser may include a mechanism that holds multiple items in storage and mechanically pushes them out discretely, one by one. Such a process and device are detailed in EP2600288, which are incorporated herein by reference

Therefore, in an embodiment, the dispensing by the conveyor and the dispensing by the supplementary item dispenser may be performed at least partially *simultaneously,* thereby mitigating or eliminating the slowness problem. In this embodiment, two container stations are provided: a first station below the end of the conveyor, for receiving the falling items, and a second station at the supplementary item dispenser, for receiving the discretely dispensed items. This way, while the supplementary item dispenser completes the missing items in a container already filled by the conveyor, other items are being dispensed into a second container at the first station, by the conveyor. When the second container is filled with the undershoot number of items, it moves to the second station for completion, and so on and so forth.

Items dispensed from a supplementary item dispenser are preferably also analysed with respect to the same characteristic, preferably color, to detect rogue or deviant items dispensed as part of the supplementary stream.

The imaging device employed for analysing the items in free-fall (e.g. the number of items that have fallen and the other characteristic(s)) may be implemented in a variety of ways. In some exemplary embodiments, a method and an arrangement may use an image sensor comprised of rows and columns of pixels.

The sensor may be controlled by a control and processing unit associated with a computing platform. The sensor may be a complementary metal-oxide-semiconductor (CMOS) sensor, a charge-coupled device (CCD) sensor or any other sensor. In preferred embodiments, the visible color of the items is analysed and the image sensor is a color image sensor, such as a Bayer-filter sensor, a Foveon X3 sensor, a 3CCD etc.

The imaging device may be a line scan color camera or an area scan color camera. For example, the sensor may be a trilinear line scan camera using three separate imaging lines to capture RGB images.

A preferred characteristic of the items to analyse is the visible color of the free-falling items. The color may in particular be associated with particular medicaments by a pharmaceutical company and may be used as a basis for rejecting counted batches including suspect or rogue items not matching a predetermined color or color spectrum. Preferably the color is identified by any one or more of hue (pure color or mix of colors), tint (mixture of pure color and white), shade (mixture of pure color and black), and/or tone (mixture of pure color with any neutral or grayscale color). The color may be determined as a spectral pattern, or as a predetermined output from a digital camera sensor (e.g. a CCD sensor or a CMOS sensor) imaging a color. For example, upon identification of a falling article, or a part of a falling article, within captured images, the output from the imaging pixels of the sensor may be compared to required signal values representing a predetermined and/or expected color. Should the sensor output not match (within acceptable tolerances) the predetermined and/or required signal values, the batch receiving the identified item may be recorded as containing a rogue or deviant item and tagged or noted for rejection.

Analysis may be done based algorithm analysis and identification of color in known color spaces.

The sensor may preferably be a digital image sensor (preferably CMOS or CCD) configured and/or adapted to operate as global shutter. This may assist in reducing external interference, skew and/or smear effects. A digital image sensor operating in global shutter mode may begin and end the exposure of the majority of, or all of, the sensor pixels simultaneously. A global shutter can allow capture of high-speed moving objects without distortion.

Exposure times for image capture are preferably minimal. This may assist in avoiding interference, skew and/or smear effects associated with image capture of fast falling.

For efficiency and speed of data processing, and given that the an expected trajectory, velocity, physical form and color of the items may be generally known in advance, it may be preferable to read and/or make use of data from only a limited number of pixels of an image sensor, for example a limited number of rows and/or columns of pixels, and/or selected colors of pixel, for example of red and green, only red and blue or only blue and green pixels.

In usual embodiments, a lens is located between the sensor and the falling area of the items, and one or more optional light sources such as white Light Emitting Diodes (LEDs) may shed light on the falling items. The LEDs may illuminate the falling area at an angle such that only minimal light is reflected from objects other than the falling items, and the lens focuses the light reflected from the falling items onto the sensor.

Since the falling speed of the items is substantially uniform due to the conveyor speed or the sliding area, the sampling rate of the sensor may be set such that light reflected from an item falling at an average speed will be captured by at least a predetermined number of consecutive sensor samples. The number of pixels affected by the item on the sensor may depend on the shape of the falling item. For example, a tablet shaped as a right circular cylinder is likely to fall such that its planar faces appear substantially as a circle. Thus, the sensor rows in which the item is seen may produce a pattern in which the first and last sensor samples in which the item is captured may contain less affected pixels than intermediate samples.

Due to the known pattern of pixels in the sensor samples affected by each tablet when it falls, and since usually items fall separately since they do not lean on each other, two or more adjacent falling items which are captured by a single sensor samples can be distinguished using previous or consecutively captured samples.

A technical effect of the disclosed subject matter is providing a method and apparatus for dispensing a predetermined number of items into a container, with high accuracy so that in almost 100 percent of the cases, the package contains exactly the required number, and the task is performed at a high efficiency so that the available resources are utilized well, and that the simultaneous analysis of at least one characteristic of each item ensures all collected items are the desired item, alternatively that rogue or deviant items are excluded, or that batches containing a rogue or deviant item can be rejected from further processing.

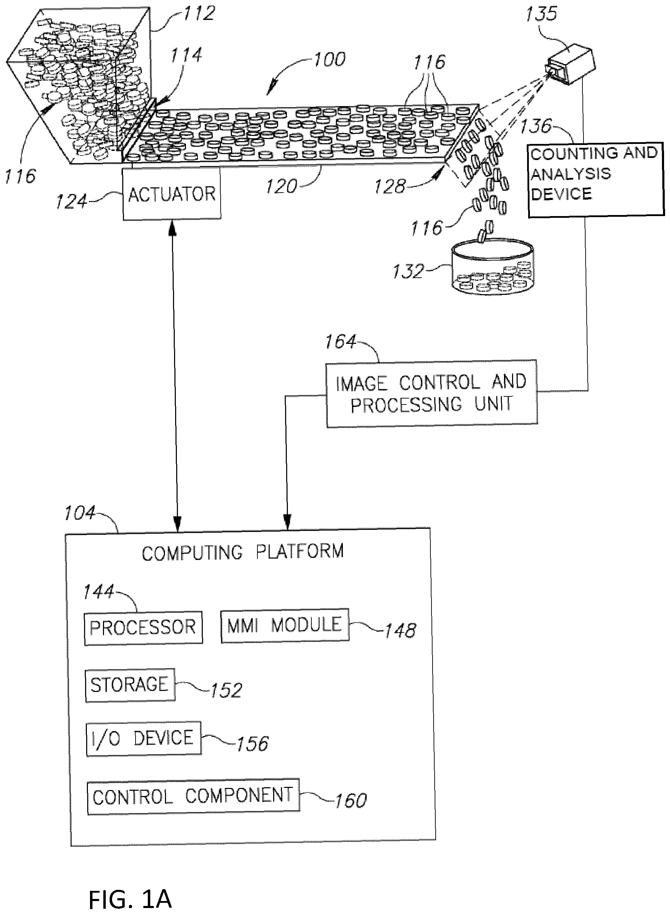

Reference is now made to Fig. 1A, which shows a schematic illustration of an apparatus for providing for dispensing and analysing one or more characteristics of a predetermined number of items at high accuracy and high efficiency.

The apparatus comprises a machine **100** communicating with and receiving control commands from a computing platform **104.** Machine **100** comprises an imaging (or "capturing") device **135** and an analysis device **136** which provides information to control and processing unit **144.** Computing platform **104** provides control commands to machine **100.**

Machine **100** comprises a reservoir, such as a hopper or silo **112,** which contains a multiplicity of items **116** to be dispensed into containers. Each container, such as container **132,** is to contain, finally, a predetermined number of items **116.**

Hopper **112,** shown here as one example of a reservoir, may comprise a gate at its lower opening **114.** Raising or lowering the gate limits the number of items **116** being dispensed from hopper **112** onto conveyor **120.** In some embodiments, lower opening 114 is wide enough to allow multiple items **116** to be dispensed onto conveyor **120** in parallel. Handling multiple items concurrently provides for fast dispensing and high yield of the method and apparatus. However, those of skill in the art will recognize that items may be dispensed onto conveyor **120** using other means known in the art.

Conveyor **120** may be a conveyor belt, a vibrating chute, a chute having variable inclination angle or the like. Optionally, conveyor **120** is of a form (hereinafter "parallel transport conveyor") which enables transporting multiple items at least partially in parallel, in a direction orthogonal to the transport direction. Conveyor **120** is of such a width that multiple items fit on its top surface in parallel.

Conveyor **120** is controlled by actuator **124,** which receives commands from computing platform **104.** Actuator **124** may operate by electrical current, hydraulic fluid pressure, pneumatic pressure or any other energy source, and converts the energy into some kind of motion applied to conveyor **120.**

The functionality of actuator **124** depends on the nature of conveyor **120.** For example, if conveyor **120** is a conveyor belt, then actuator **124** drives or stops the belt; if conveyor **120** is a vibratory chute then actuator **124** starts or stops a vibration engine; if conveyor **120** is a variable inclination chute then actuator **124** lowers or raises one side of the chute, or the like.

In some embodiments, actuator **124** causes vibrations in conveyor **120,** which cause the items on conveyor **120** to assume a certain position. For example, if the dispensed items are cylinder-shaped medicinal tablets, the vibrations may cause them to assume a position on conveyor **120** such that one of their planar sides rests on conveyor **120,** and generally no item leans partially or fully on another item. However, the items may occasionally touch each other.

Items **116** proceed along or with conveyor **120** when operated, until the conveyor's end **128.**

From the conveyor's open end **128,** the items free-fall and reach container **132,** either directly, as shown, or through a track or other processing path (not shown) that leads to the container.

In some embodiments, the speed of actuation of conveyor **120** and therefore the speed of the items placed thereon, can be for example between about 2 cm per second and 20 cm per second, e.g., 6 cm per second.

The falling items are imaged by capture device **135** as they travel through a virtual "imaging area" **129** extending from conveyor end **128** or slightly below it to a certain distance below, forming a virtual convex rectangle which faces the capture device. The images may then be analyzed by analysis device **136.**

Analysis device **136** may receive control commands, such as a command to sample a sensor within capture device **135,** from control and processing unit **164.** Control and processing unit **164** may be a part of, or otherwise associated with computing platform **104** detailed below. Analysis device **136** may provide the imaged data to control and processing unit **164** for counting and analysis of other characteristics.

Control and processing unit **164** may transfer the raw data or the counting results or intermediate results to computing platform **104.**

In some embodiments, control and processing unit **164** may be implemented as part of computing platform **104,** for example as an application executed by computing platform **104.** However, in other embodiments, control and processing unit **164** may be implemented separately from computing platform **104,** or as a part of analysis device **136.**

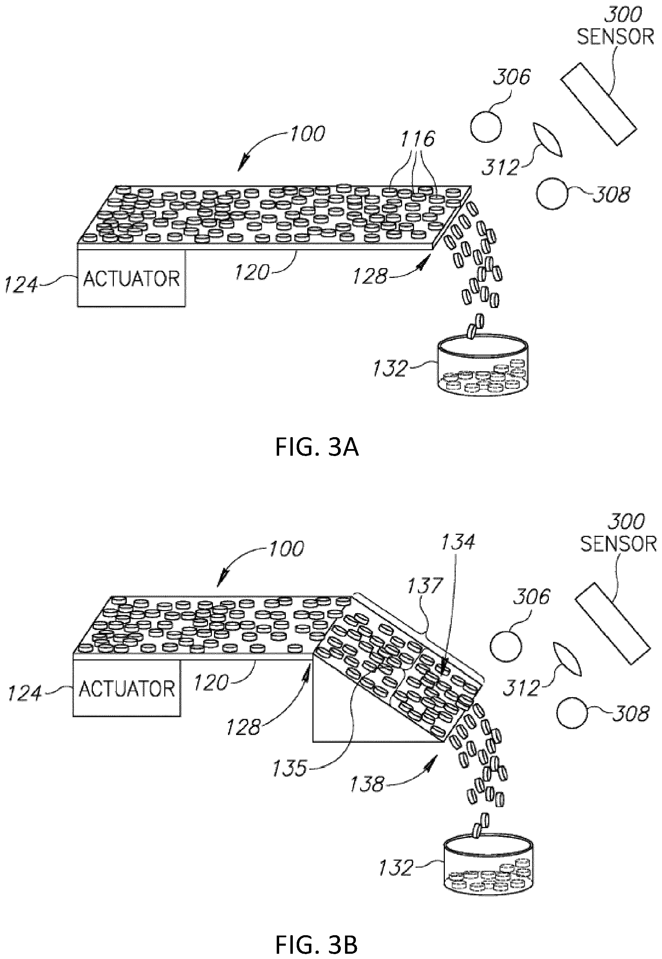

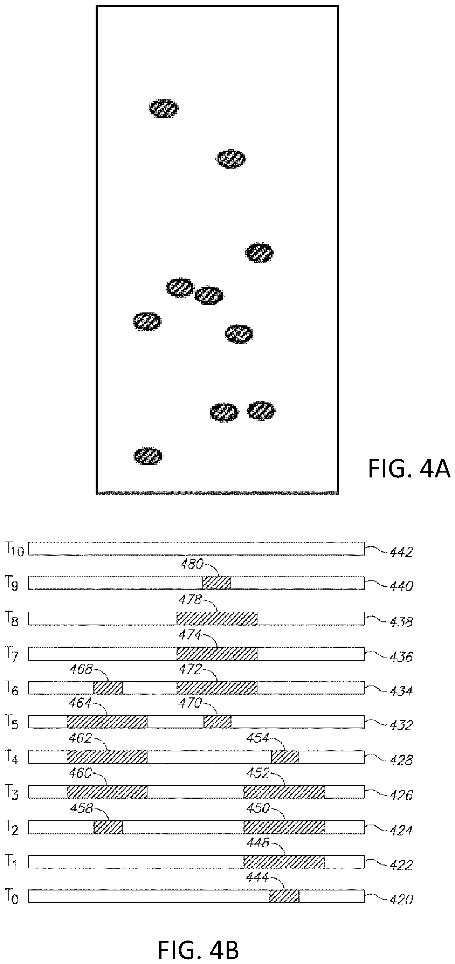

Analysis device **136** and the operation of control and processing unit **164** is further detailed in association with Fig. 3 and Figs. 4A-4B below.

Computing platform **104** may comprise a processor **144.** Processor **144** may be any Central Processing Unit (CPU), a microprocessor, an electronic circuit, an Integrated Circuit (IC) or the like. Alternatively, computing platform can be implemented as hardware or configurable hardware such as field programmable gate array (FPGA) or application specific integrated circuit (ASIC). In yet other alternatives, processor **144** can be implemented as firmware written for or ported to a specific processor such as digital signal processor (DSP) or microcontrollers. Processor **144** may be used for perfoming mathematical, logical or any other instructions required by computing platform **104** or any of it subcomponents.

In some embodiments, computing platform **104** may comprise an MMI (man-machine interface) module **148.** MMI module **148** may be utilized for receiving input or providing output to and from machine **100,** analysis device **136,** or a user, for example receiving specific user commands or parameters related to calibrating and operating the apparatus, storing and retrieving information, providing output for analyzing performance of the apparatus, or the like.

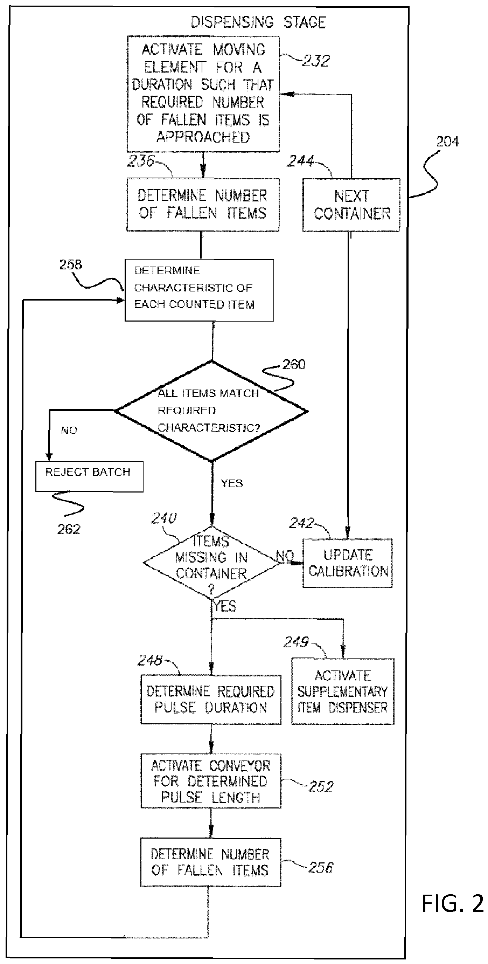

In some exemplary embodiments, computing platform **104** may comprise one or more storage devices such as storage device **152.** Storage device **152** may be non-transitory (non-volatile) or transitory (volatile). For example, storage device **152** can be a Flash disk, a Random Access Memory (RAM), a memory chip, an optical storage device such as a CD, a DVD, or a laser disk; a magnetic storage device such as a tape, a hard disk, storage area network (SAN), a network attached storage (NAS), or others; a semiconductor storage device such as Flash device, memory stick, or the like. In some exemplary embodiments, storage device **152** may retain program code of control component **160** detailed below operative to cause processor **144** to perform acts associated with any of the steps of Fig. 2 detailed below, displaying information to the user, or the like. Storage device **152** may also retain information such as calibration results to be used when operating the machine for a particular type of dispensing task, number of finished containers, the number of items in each container, container identification of containers containing deviant or rogue items, or the like.

Computing platform **144** may further comprise or be associated with one or more Input/Output (I/O) devices **156** communicating with MMI module **148,** such as a terminal, a display, a keyboard, an input device or the like, to interact with the system, to provide instructions for calibrating the machine or the like.

Computing platform **144** may also execute control component **160** for determining and generating control commands to be provided to actuator **124,** optionally during calibration, and optionally during operation, for example in accordance with counts received from analysis device **136.**

Control component **160** can be implemented as one or more sets of interrelated computer program instructions, which may be developed using any programming language and under any development environment. The computer program instructions may be stored on storage **152** and provided to processor **144** or any other programmable processing apparatus to produce a machine, such that the instructions, which execute via the processor, create means for implementing the functions specified in the flowcharts or block diagrams.

The computer program instructions may also be stored on a computer-readable non-transitory medium. The steps performed by control component **160** are further detailed in association with Fig. 2 below.

It will be appreciated that computing platform **144** can be provided remotely from machine **100,** as part of machine **100,** or in any combination thereof.

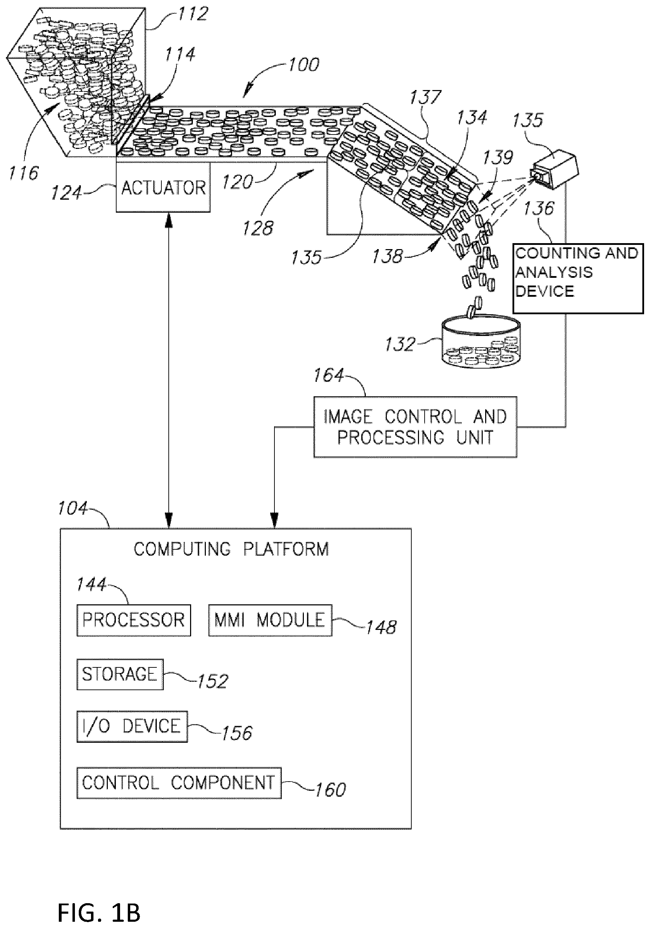

Reference is now made to Fig. 1B, which shows a schematic illustration of another embodiment of an apparatus for providing for dispensing predetermined number of items at high accuracy and high efficiency.

As in Fig. 1A, the apparatus comprises machine **100,** hopper or silo **112** with a gate at its lower opening **114,** conveyor **120,** actuator **124,** computing platform **104,** capture device **135,** analysis device **136,** and container **132.**

Items **116** are released onto conveyor **120,** and proceed along or with conveyor **120** when operated, until the conveyor's end **128.**

From conveyor end **128,** the items fall onto and along slope **134.** It will be appreciated that slope **134,** in some embodiments, may be made of a rough material so that the items do not accelerate along slope **134,** but their speed is maintained due to the friction. However, the friction coefficient between slope **134** and the items is such that the friction does not hold the items from sliding, and does not cause the items to roll, thus keeping unchanged the side of the item that touches slope **134.**

Slope **134** causes the items to assume a substantially uniform speed as they fall off end **138** of slope **134,** as well as continue their free-fall at a similar trajectory, such that their angle in relation to capture device **135** is similar.

In some embodiments, the speed of conveyor **120** and therefore the speed of the items placed thereon, can be for example between about 2 cm per second and 20 cm per second, e.g., 6 cm per second. The speed of the items sliding along slope **134** may increase relatively to the speed of conveyor **120** and may get to between about 20 cm per second and about 2 meters per second, depending on the material of slope **134** and its angle.

At end **138** of slope **134,** the items free-fall and reach container **132.**

Capture device **135** is positioned and set so as to capture images of the items as they fall off end **138** of slope **134,** unlike the setting of Fig. 1A at which the items are captured as they travel along a virtual "imaging area" **139** extending from end **138** of slope **134** or slightly below it to a certain distance below, forming a virtual convex rectangle which faces the capture device. The images are analyzed and machine **100** is controlled and activated in substantially the same manner as in the embodiment of Fig. 1A.

Referring now to Fig. 2, showing a flowchart of steps for operating a dispensing machine, such as the one shown in Fig. 1, to provide high accuracy and high efficiency dispensing and analysis of items, thus yielding high throughput and qualitative accuracy.

In step **232,** the conveyor is activated for a period of time determined such that the number of items falling due to activation approaches, but doesn't reach, the number of items it is required to dispense in each container. The duration is determined in accordance with the first throughput function determined on step **216** of the calibration stage. In some embodiments, the period of time is determined such that in the majority of cases, the container will contain fewer than the required number of items. The reasoning for that is that it is generally desired to have fewer items, which is correctable by adding items, than having too many items dispensed.

In step **236,** the number of items that have fallen into the container is determined. The number of items also includes the items that have fallen due to inertial forces after the conveyor has stopped. It will be appreciated that in some embodiments the items are counted as they fall, which happens when the conveyor is in motion and some time afterwards.

In step **258,** a characteristic, preferably a color, of every counted item is determined based on captured image data.

In step **260,** it is determined whether every counted item in a batch matches a required or predetermined characteristic, preferably a specified color characteristic. If not all counted items meet the required or predetermined characteristic, the batch of counted items is rejected at step **262.**

If all counted items meet the required or predetermined characteristic, in step **240** it is determined whether items are still missing in the container to complete the entire quantity that has to be dispensed.

If no items are missing, which may be a rare occasion, then on optional step **242,** the throughput functions or parameters thereof as set on calibration steps **200,** such as the values of particular points in the throughput functions, are updated based on the number of items that have fallen during the initial operation and the one or more pulses as discussed in patent publication EP2600288. Whether the calibration parameters have been updated on the fly or not, the container is removed, and the next container is placed on step **244.**

If items are still missing, two options are available, in two embodiments or in a unified embodiment: In the first option, in step **248,** the required duration is determined for a pulse length, such that the items that will fall due to the pulse will approach or complete the required number of items.

Thus, in step **252** the conveyor may be activated for the determined or predetermined pulse length.

In step **256** the number of fallen items is determined similarly to step **236** above, and control returns to step **258.**

Depending on the usage and nature of the items to be dispensed, in some embodiments, a single activation of the conveyor would be enough to ensure that in large enough percentage of the cases, the number of dispensed items is within satisfactory range from the required number. If, however, greater accuracy is required, then one or more pulses would be required to achieve the goal so that no over shooting occurs.

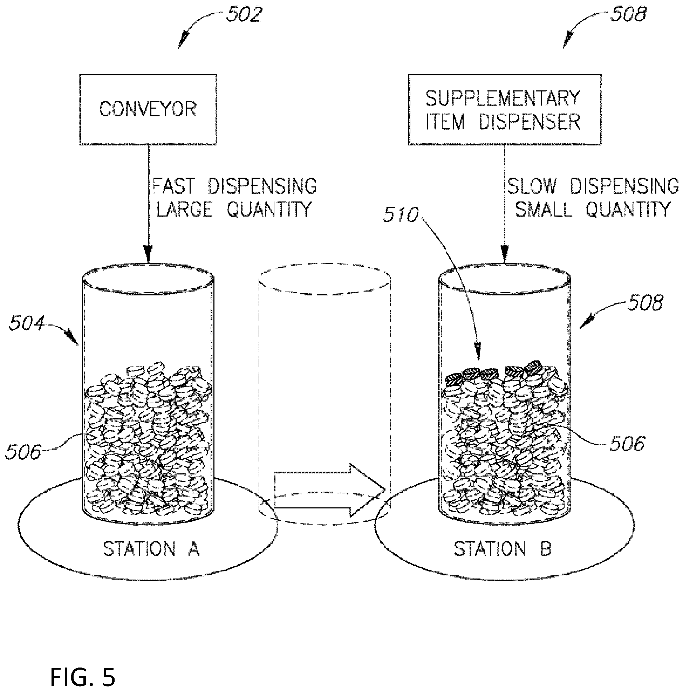

In the second option employed when items are still missing in step **240,** a supplementary item dispenser may be utilized to dispense the missing items. Interim reference is now made to Fig. 5, which depicts this option. Firstly, a conveyor **502** is used to rapidly dispense a relatively large number of items, for example tens, hundreds or thousands of items in one operation. Conveyor **502** is stopped as the number of items **506** in a container **504** almost reaches the desired final number, such that even after the inertial fall, a few items (for example 1-10) will still be missing. Container **504** is then transported, using automatic means, from station A near the conveyor to station B near a supplementary item dispenser **508.** Supplementary item dispenser **508** then dispenses the missing items **510** into container **504,** typically at a much slower rate than the throughput of conveyor **502.** While supplementary item dispenser **508** operates, conveyor **502** is operated again, to fill another container. This process continues until the desired amount of containers have been filled to completion. The items dispensed by the supplementary dispenser **508** are similarly checked for the required characteristic, and the batch is rejected in the event any of the missing items **510** do not meet the required characteristic.

Reference is now made to Fig. 3A, showing an embodiment of a capturing device as used with the embodiment of Fig. 1A above. The capturing device captures images of items such as items **116** as they fall off end **128** of conveyor **120.** The items **116** are shown being dispensed in bulk (a 'water-fall' type dispensing as opposed to single-file) with multiple items **116** falling in parallel and adjacent in height while falling.

The capturing device **135** comprises sensor **300,** which may be a CMOS, a CCD or any other imaging sensor. The sensor may be implemented as a one- or two-dimensional collection of pixel sensors, each pixel containing a photodetector and an amplifier. If sensor **300** comprises a two-dimensional array, the rows or columns of the pixel sensors may or may not be aligned. The sensor may be a color image sensor, such as a Bayer-filter sensor, a Foveon X3 sensor, a 3CCD, a line scan color camera, a trilinear line scan sensor using three separate imaging lines to capture RGB images, or an area scan color sensor.

The sensor may be positioned such that its face is at an angle of between about 45 degrees and about 135 degrees, e.g. 90 degrees to a tangent to the trajectory of the falling items, at the virtual imaging area.

Sensor **300** may be located such that its central area, e.g., one or more central rows, are closest to end **128** of conveyor **120.** In some exemplary embodiments, the central area of sensor **300** may be located at a distance of between about 20 cm and about 40 cm from the central area of end **128.**

Capturing device **135** may further comprise one or more light sources such as light sources **306** and **308** which shed light constantly or intermittently on the falling items.

The sensor **300** may preferably be a digital image sensor (preferably CMOS or CCD) configured and/or adapted to operate with as a global shutter. This may assist in reducing external interference, skew and/or smear effects. A digital image sensor operating in global shutter mode may begin and end the exposure of the majority of, or all of, the sensor pixels simultaneously. A global shutter can allow capture of high-speed moving objects without distortion. A rolling shutter may alternatively be used but it is more susceptible to distortion effects for high speed objects.

Exposure times for image capture of items **116** as they fall off end **128** are preferably minimal. This may assist in avoiding interference, skew and/or smear effects associated with image capture of fast falling items **116.** The lux of the light sources is suitably high to allow short exposure times.

Light sources such as light sources **306** and **308** may preferably provide full spectrum white light, although partial spectrums may also be provided for specifically expected colors of items or possible rogue or deviant items.

In embodiments wherein light is shed intermittently, the pulse length and frequency of the LEDs may be selected such that enough light will be reflected from each item to activate the sensor.

Light sources **306** and **308** are optionally positioned such that light will not be reflected or only minimally reflected from other objects except the items that have fallen off end **128.**

Capturing device **135** may further comprise a lens **312** for focusing the light reflected from the falling items onto sensor **300.** Lens **312** is optionally a lens assembly, which may, in some embodiments, provide also an optical zoom function.

The images sampled by sensor **300** may be transferred to image control and processing unit **164,** which may perform the counting and analysis of the characteristic, preferably the color output from the sensor, and report the number and characteristic information to computing platform **144.** The measured characteristic, such as color, may be compared to a required or expected characteristic by the processing unit **164,** and the batch tagged or noted as a bad-batch or rejection-batch if one or more items deviate (beyond a tolerance level) from the required or expected characteristic e.g. color. Alternatively the raw sensor data may to passed to the computing platform **144** for processing.

Reference is now made to Fig. 3B, showing an embodiment of capturing device **135** as used with the embodiments shown in Fig. 1B above. As in Fig. 3A above, capturing device **135** comprises a sensor **300,** light sources **306** and **308,** and lens **312.** However, sensor **300** is positioned such that it captures images of items as they fall off end **138** of slope **134.**

Sensor **300** may be located such that its central area, e.g., one or more central rows, are closest to end **138** of slope **134.** In some exemplary embodiment, the central area of sensor **300** may be located at a distance of between about 20 cm and about 40 cm from the central area of end **138.**

Due to inertial forces, items fall off end **138** in substantially the same posture in which they slid along slope **134.** Thus, sensor **300** may be substantially parallel to a planar face of items falling off end **138** of slope **134,** and substantially parallel to the continuation of the plane of slope **134.**

Light sources **306** and **308** may shed light constantly or intermittently on the falling items, and may be positioned such that light will not be reflected or only minimally reflected from slope **134** but only from the items that have fallen off end **138,** such as item **118.**

On the conveyor **120** the items generally assume a stable posture and do not lean on one another, together with their speed being substantially uniform, only in rare cases are items imaged as partially or fully overlapping. As multiple items fall off the end **128** of conveyor **120** in the embodiment of Fig. 1A, or off end **138** of slope **134** in the embodiment of Fig. 1B, they may disperse from one another into a waterfall type distribution. For example, even if two or more items are adjacent to one another, they can be separate when they tip over the edge and the gaps between the items increase. This distribution of items may be imaged.

The falling items 116 may preferably be imaged using a sensor operated as a global shutter. That is each pixel used for imaging, preferably all imaging pixels of the sensor, begins and ends the exposure simultaneously. This can advantageously capture an image of the rapidly falling items without distortion. An image obtainable via global shutter imaging is exemplified in Fig. 4A. The image contains data taken for all imaging sensor pixels taken at a time T₀. Each item in a global shutter can be counted and one or more characteristics, preferably at least color, can be assessed for each item in the image.

Alternatively, although a global shutter may be preferred, a rolling shutter image capture may also be implemented in which relatively narrow horizontal slices of an image of each falling item are detected. This is exemplified in Fig. 4B, showing a consecutive sequence of samples of a sensor line. One or more lines of a sensor may be utilized. Hence, the case shown in this figure is the most general one, which shows only a single line of pixels. Sample **420,** taken at time T₀ comprises a narrow sequence **444** of pixels affected by a lower edge of a first item consisting of sequences **444, 448, 450, 452** and **454,** while samples **422, 424, 426** and **428,** taken at times T₁, T₂ and T₃, respectively, show wider sequences **448, 450,** and **452** of item **408,** respectively, and sample **428** taken at time T₄ also shows again a narrow sequence **454** of pixels affected by item **408.**

Similarly, sequences **458, 460, 462, 464** and **468** of samples **424, 426, 428, 430** and **432,** respectively, taken at times T₂, T₃, T₄, T₅ and T₆ respectively, wherein the sequences are created by the light reflected from a second item, and sequences **470, 472, 474, 478** and **480** of samples **432, 434, 436,** 438 and **440,** respectively, taken at times T₅, T₆, T₇, T₈ and T₉ respectively, wherein the sequences are created by the light reflected from a third item.

Using the known pattern of affected pixels created by a falling item on consecutive capturing of a sensor line, the sequence of sensor line samples taken at times T₀ to T₉ are analyzed, and the three items are detected and counted. For example, distinguishing and counting the right cylindrical shapes may be based on the lower number of affected pixels at their end samples, and higher number at intermediate samples.

It will be appreciated that even if items may fall at different speeds, the general pattern may still be valid although it may slightly change. For example, if an item falls at a higher speed, its central part may appear in fewer than three samples, or its narrower first or last sample may be missing. Thus, in some embodiments, the pattern may be searched for with some flexibility.

It will also be appreciated that if two or more items seem adjacent on the sensor, the number of items may be determined by dividing the number of affected pixels by the maximal estimated number of pixels affected by a single item, and rounding the result to the higher number. Some measurements have shown that in about 1 percent of the cases two items may seem adjacent, while in significantly smaller percentage three or more items are adjacent, therefore such division may provide satisfactory results.

Each line of pixels in a global shutter or rolling shutter sensor may include pixels for sensing red, green and/or blue, or alternating pixel lines may sense green and blue, and red and green incident light, such as in a Bayer-filter.

In addition to noting a positive point of reflection from an item, the sensor information may thus also provide information indicative of the item color through the varying signal levels for each of the variously colored pixels. The color output of one or more pixel lines may be compared to a required or expected exemplary sensor signal (possibly calibrated for a given product in situ, or factory preprogramed) that is stored by the processing unit **164** or computing platform **144.** Deviation from the required or expected exemplary sensor signal values for a counted item may be used as the basis for rejection of a batch of counted items.

Using a single sensor, and sampling a single sensor line, or a number of sensor lines, such as 20 sensor lines, provides for cost reduction, as well as efficient detection of the falling items. The efficient detection can be used for increasing the detection speed, thus enabling the counting of more items per time unit. In other embodiments, however, two or more sensors may be used. In some preferred embodiments, all pixels may be captured simultaneously as in global shutter imaging.

The analysis of the sensor output can be performed by control and processing mechanism **164** of Fig. 1, or by computing platform **104.**

In some further analysis, image analysis techniques may also be used for determining whether a falling item is whole or broken, according to its various projections on the sensor. If this feature is provided, broken items can be either ignored or removed from the item stream so that the container will comprise at least the required number of proper items. Alternatively, the entire packaged unit **132** may be discarded. Broken items may also be identified based on a deviation in color from an expected color, for example when handling coated articles, a differently colored interior (e.g. white) may be revealed to the sensor and used as a basis for determining the presence of a deviant item.

The invention has been described by reference to certain embodiments discussed above. It will be recognized that these embodiments are susceptible to various modifications and alternative forms well known to those of skill in the art without departing from the spirit and scope of the invention. Accordingly, although specific embodiments have been described, these are examples only and are not limiting upon the scope of the invention, which is defined in the accompanying claims.

## Claims

1. A method comprising:
- allowing items to fall into a volume;
- employing a digital imaging device to acquire images of the items while said items are falling;
- processing the images to count each item;
- processing the images to determine at least one characteristic, preferably a color, of each item; and
- evaluating said determined characteristic in relation to at least one predetermined characteristic.

2. The method according to claim 1 wherein the acquired camera images are acquired with a high-speed camera, preferably the camera is arranged to acquire at least 150 images per second, preferably at least 200 images per second, and more preferably at least 250 frames per second.

3. The method according to claim 1 or 2 wherein the method dispenses batches of predetermined numbers of items, and batches are rejected from further processing if one or more inspected free-falling items in a batch has a characteristic deviant from a required characteristic.

4. The method according to any of claims 1 to 3, wherein acquired images are color images, and wherein the determined characteristic is a visible color of the items and the predetermined characteristic is a visible color, preferably when the determined and predetermined characteristics are color hue.

5. The method according to any of claims 1 to 4, wherein the items fall in free-fall, and preferably wherein items in free-fall are spaced from any solid background surface, preferably by at least 2 mm, more preferably at least 5 mm.

6. The method according to any of claims 1 to 5, wherein the items fall in front of a colorless, black or grey background.

7. The method according to any of claims 1 to 6, wherein the objects are medicinal tablets, pills or capsules.

8. The method according to any of claims 1 to 7, wherein the items are arranged in a single layer upon a conveyor and at least some of the items are transported in parallel upon the conveyor.

9. The method according to any of claim 1 to 8 wherein the images are processed in real-time, to continuously determine the number of falling items.

10. The method according to any of preceding claims 1 to 9, further comprising the steps of:
- providing a conveyor with an open dispensing end; and
- transporting the items in bulk on the conveyor to the open dispensing end of the conveyor;
preferably wherein falling items are imaged while falling through a volume substantially immediately below the open end of the conveyor.

11. An apparatus comprising:
a dispenser comprising an open dispensing end, configured to dispense a plurality of items;
a volume below the open dispensing end;
at least one digital imaging device positioned to capture images of items falling from the open dispensing end;
a processer configured to analyse images captured by the digital imaging device, said analysis comprising at least a count of the number of items, and a determination of at least one characteristic, preferably color, of each imaged item.

12. The apparatus according to claim 11, wherein the digital imaging device is configured to acquire color images and the processor is configured to determine a color of each imaged falling item and to evaluate said color in relation to at least one predetermined color.

13. The apparatus according to claim 11 or 12, wherein the imaging device comprises one or more digital cameras, said cameras being arranged to acquire at least 150 images per second, preferably at least 200 images per second, and more preferably at least 250 frames per second.

14. The apparatus according to any of the claims 11 to 13, further comprising one or more light sources to illuminate falling items.

15. The apparatus according to any of claims 11 to 14, further comprising a rejection conduit for ejection of items identified as deviant from a required characteristic, or batches of items that contain items identified as deviant from a required characteristic.

16. Computer readable medium having computer readable instructions stored thereon for performing, when executed by a processor of an apparatus according to any of claims 11 to 15, the method as defined by any of claims 1 to 10.
